## Europäisches Patentamt

## European Patent Office

## Office européen des brevets

(19)

(11) Veröffentlichungsnummer: **0 049 518**

**A1**

(12) **EUROPÄISCHE PATENTANMELDUNG**

(21) Anmeldenummer: **81107987.0**

(22) Anmeldetag: **06.10.81**

(51) Int. Cl.³: **A 61 B 10/00**
**G 01 N 29/00**

(30) Priorität: **08.10.80 DE 3038052**

(43) Veröffentlichungstag der Anmeldung:
**14.04.82 Patentblatt 82/15**

(84) Benannte Vertragsstaaten:
**AT CH DE FR GB LI NL**

(71) Anmelder: **SIEMENS AKTIENGESELLSCHAFT Berlin
und München**
**Postfach 22 02 61**
**D-8000 München 22(DE)**

(72) Erfinder: **Maderlechner, Gerd, Dipl.-Phys.**
**Arnika-Strasse 5**
**D-8031 Gröbenzell(DE)**

(72) Erfinder: **Trautenberg, Elmar, Dr. rer. nat.**
**Steinacher-Strasse 32a**
**D-8510 Fürth(DE)**

(54) Verfahren zum Abtasten eines Objekts zu dessen Abbildung mittels Ultraschall-Echo-Tomographie.

(57) Das Verfahren verwendet eine Vorrichtung mit zumindest einem Ultraschallwandler (3) mit schmaler Schallkeule (31), die an sich jeweils nur ein Gebiet eines abzubildenden Objekts (1) erfassen kann. Das gesamte Objekt (1) wird jeweils durch mehrere Abtastvorgänge abgedeckt, wozu die Schallkeule nacheinander schrittweise quer zu ihrer Ausbreitungsrichtung einen lateralen oder sektorförmigen Weg beschreibt. Die sich aus den einzelnen Schritten ergebenden Echofolgen werden analog oder digital mittels eines Rechners aufsummiert und bilden jeweils eine Projektion im Sinne der Ultraschall-Echo-Tomographie.

FIG 1

EP 0 049 518 A1

SIEMENS AKTIENGESELLSCHAFT
Berlin und München

Unser Zeichen
VPA    80 P 7 5 7 5 E

Verfahren zum Abtasten eines Objekts zu dessen Abbildung  mittels Ultraschall-Echo-Tomographie

Die vorliegende Erfindung betrifft ein Verfahren zum
Abtasten eines Objekts zu dessen Abbildung mittels
Ultraschall-Echo-Tomographie und darüber hinaus eine
Abtastvorrichtung zur Durchführung dieses Verfahrens.

Ultraschallwandler weisen in aller Regel eine endliche
Schallkeule auf. Bei ihrer Anwendung auf dem Gebiet der
Ultraschall-Echo-Tomographie, die ein Objekt in Analogie zu der bekannten Röntgen-Tomographie abbildet, ist
die mit der genannten Schallkeulenbreite verbundene
Richtwirkung störend, vergl. beispielsweise G.Wade et
al:"Acoustic Echo Computer Tomography", Acoustical
Imaging, Vol. 8, A.F. Metherell (ed.), Plenum 1980.

Da das abzubildende Objekt vollständig mit Ultraschallwellen abzudecken ist, wurden bei bekannten Tomographie-
Verfahren, die auf dem Ultraschallprinzip beruhen,
bisher breite, ebene Piezoschwinger, vergl. ebenfalls
o.a. Literaturstelle, verwendet. Derartige Verfahren
bzw. Anordnungen haben jedoch Mängel. So liegt das
Objekt im Nahfeld des Schwingers, wodurch in aller
Regel Verstärkungen und Auslöschungen der Echosignale
infolge von Interferenzen auftreten, was eine Verfälschung der Objektabbildung zur Folge haben kann. Die
Impulsform eines derartigen Schwingers ist daher wesentlich ungünstiger als die eines Schwingers mit kleiner
Fläche. Kleine Schwinger von der Größenordnung einer
Wellenlänge (0,5 mm) haben den Nachteil einer geringen
Empfindlichkeit. Sie sind deshalb trotz der Tatsache,
daß sie eine ausreichend breite Schallkeule aufweisen,
wenig geeignet.

Pap 1 Ne/07 10 1980

Der vorliegenden Erfindung liegt die Aufgabe zugrunde, ein Verfahren zum Abtasten eines Objekts zu dessen Abbildung mittels Ultraschall-Echo-Tomographie zu schaffen, bei dem Ultraschallwandler mit schmaler Schallkeule zur Anwendung gelangen, die einen kurzen, stark abklingenden Impuls erzeugen und somit wesentlich besser als Ultraschallwandler der zuvor genannten Art für eine Anwendung in der Ultraschall-Echo-Tomographie geeignet sind.

Die der Erfindung zugrundeliegende Aufgabe wird durch ein Verfahren zum Abtasten eines Objekts zu dessen Abbildung mittels Ultraschall-Echo-Tomographie gelöst, das dadurch gekennzeichnet ist, daß das abzubildende Objekt jeweils aus verschiedenen Einstrahlrichtungen mittels zumindest eines Ultraschallwandlers, der eine schmale, den jeweils abzutastenden Bereich des Objekts nur teilweise erfassende Schallkeule aufweist, schrittweise nacheinander aus einer Vielzahl von Abtastpositionen, nämlich nach Art eines Scan-Vorganges, abgetastet wird, wobei der Wechsel der Abtastpositionen quer zu der Schallkeule orientiert ist, und daß die Echofolgen aus jeder der Positionen eines solchen Scan-Vorganges aufsummiert werden und somit die Wirkung einer breiten Schallkeule erreicht wird.

Die Erfindung bietet den Vorteil, daß ein gegenüber bekannten derartigen Verfahren verbessertes Verfahren durch Verwendung besser geeigneter Ultraschallwandler geschaffen worden ist.

Vorteilhafte Weiterbildungen der Erfindung sind durch die in den Unteransprüchen angegebenen Merkmale gekennzeichnet.

.Im folgenden wird die Erfindung an Hand mehrerer,
Ausführungsbeispiele für die Erfindung betreffender
Figuren im einzelnen erläutert.

Fig. 1 zeigt schematisch das der vorliegenden Erfindung zugrundeliegende Prinzip einer Objektabtastung, bei der ein Objekt 1 aus verschiedenen Einstrahlrichtungen 2,2',2"... mittels
zumindest eines Ultraschallwandlers 3 mit einer
schmalen Schallkeule 31 abgetastet wird.
(Das Abtastverfahren ist durch einen Doppelpfeil angedeutet).

Fig. 2 zeigt schematisch eine erfindungsgemäße Verschiebeanordnung 4, die den Ultraschallwandler 3 trägt und mittels derer der Ultraschallwandler 3 jeweils aus einer Abtastrichtung,
z.B. 2, schrittweise relativ zu dem abzutastenden Objekt 1 verschiebbar ist, sowie einen
Rechner 9.

Fig. 3 zeigt schematisch eine erfindungsgemäße Reihenanordnung mit einer Vielzahl von Ultraschallwandlern $3_1...3_n$, die zeitlich nacheinander
mittels einer Steuereinrichtung 8 aktivierbar
sind, sowie einen Rechner 9.

Fig. 4 zeigt schematisch eine erfindungsgemäße Schwenkanordnung für einen Ultraschallwandler 3,
mittels derer ein abzutastendes Objekt 1 über
die Schallkeule 31 sektorförmig abgetastet
werden kann.

Fig. 5 zeigt schematisch eine erfindungsgemäße Reihenanordnung mit einer Vielzahl von Ultraschallwandlern $3_1'...3_n'$, die auf einem bogenförmigen Tragkörper 6 derart befestigt sind, daß die einzelnen Ultraschallkeulen $31_1'...31_n'$ radial zueinander ausgerichtet sind, wobei die Ultraschallwandler $3_1'...3_n'$ zeitlich nacheinander durch eine Schaltungsanordnung 5 aktivierbar sind, sowie einen Rechner 9.

Fig. 6 zeigt schematisch eine erfindungsgemäße Reihenanordnung mit einer Vielzahl von Ultraschallwandlern $3_1''...3_n''$, die auf einem ebenen Tragkörper 7 befestigt sind, wobei die einzelnen Ultraschallwandler $3_1''...3_n''$ mittels einer abgewandelten Schaltungsanordnung 5' jeweils in ihrer Gesamtheit, jedoch mit Impulsen verschiedener Phasenlagen derart erregbar sind, daß sich die jeweils erforderliche Strahlrichtung der entstehenden Gesamtschallkeule ergibt, sowie einen Rechner 9.

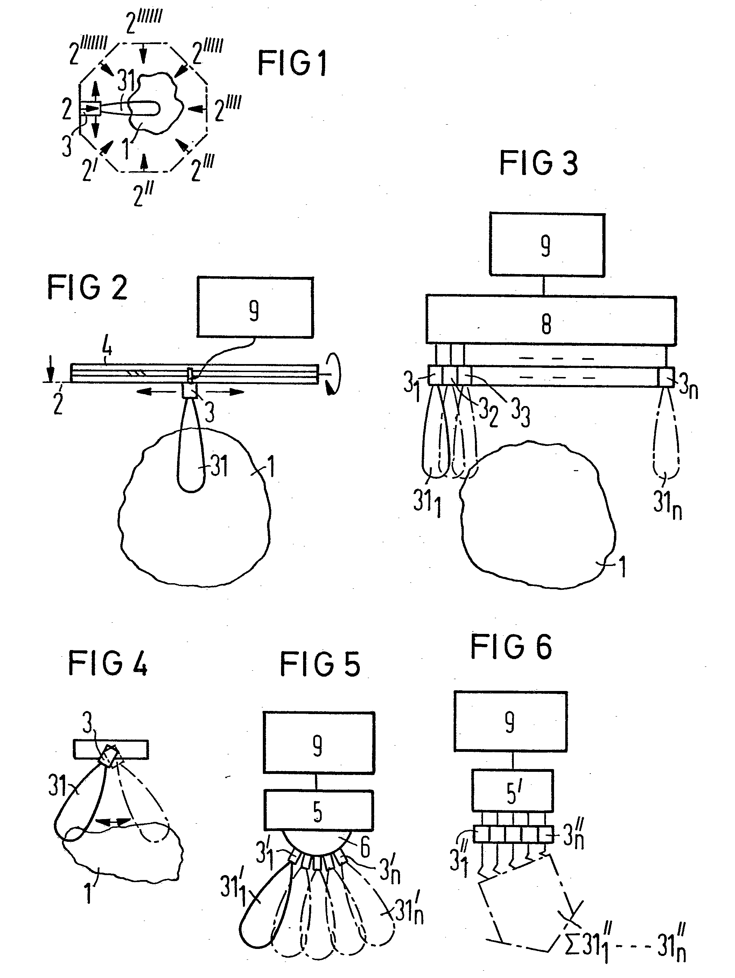

Wie bereits erläutert, zeigt Fig. 1 schematisch das der vorliegenden Erfindung zugrundeliegende Prinzip einer Objektabtastung, bei der ein Objekt 1 aus verschiedenen Abtastrichtungen 2,2',2''... mittels zumindest eines Ultraschallwandlers 3 mit einer schmalen Schallkeule 31 abgetastet wird. Die Echofolgen aus jeder der Abtastpositionen quer zur Schallkeule werden erfindungsgemäß beispielsweise mittels eines Rechners (in Fig. 1 nicht gezeigt) analog oder digital aufsummiert. Vor ihrer Summation werden die Echofolgen in an sich bekannter Weise entweder analog (mit Hilfe eines Gleichrichters und ggf. zusätzlicher Glättungsmittel) oder digital, beispielsweise mittels eines Rechners, gleichgerichtet, um Interferenzstörungen, die die Objektabbildung verfälschen könnten, auszuschließen. Durch die Summation wird im übrigen das Signal/Stör-Verhältnis verbessert.

Der erforderliche Wechsel der Abtastpositionen quer zu der Schallkeule erfolgt erfindungsgemäß entweder durch eine laterale Bewegung der Schallkeule oder durch eine Schwenkbewegung der Schallkeule.

Wie bereits erläutert, zeigt Fig. 2 schematisch eine erfindungsgemäße Verschiebeanordnung 4, die den Ultraschallwandler 3 jeweils in einer Abtastebene 2 oder 2' oder 2" usw. schrittweise relativ zu dem abzutastenden Objekt verschieben kann. Die Schallkeule 31 beschreibt dabei jeweils einen lateralen Weg über das abzutastende Objekt 1. An den Ultraschallwandler 3 ist zur Auswertung in an sich bekannter Weise ein Rechner 9 angeschlossen. Die Verschiebeanordnung 4 besteht vorzugsweise aus einer Laufschiene, in der eine motorgetriebene Spindel drehbar gelagert ist, mit der ein Tragkörper für den Ultraschallwandler 3 in wirksamem Eingriff zu einer Verschiebung längs der Laufschiene steht.

Eine vorteilhafte Weiterbildung der Erfindung sieht vor, daß zu dem Wechsel der Abtastpositionen quer zu der Schallkeule eine Vielzahl von in Reihe angeordneten Ultraschallwandlern $3_1 \ldots 3_n$ vorgesehen ist und daß die einzelnen Ultraschallwandler $3_1 \ldots 3_n$ dieser Reihenordnung in an sich bekannter Weise zeitlich nacheinander aktiviert werden. Dazu ist eine Anordnung gemäß Fig. 3 vorgesehen.

0049518

Wie erkennbar, ist eine Steuereinrichtung 8 angeordnet, die individuelle elektrische Verbindungen zum Ansteuern der einzelnen Ultraschallwandler aufweist, über die die Ansteuerung vorgenommen werden kann.

Das jeweilige Ein- bzw. Ausschalten der einzelnen Ultraschallwandler kann beispielsweise durch individuelle rechnergesteuerte Thyristoren vorgenommen werden. Zum Auswerten der Echofolgen, insbesondere zum Aufsummieren, ist an die Steuereinrichtung ein Rechner 9 angeschlossen. Eine Anordnung gemäß Fig. 3 bietet den Vorteil, daß zum Ausführen der lateralen Bewegung der Schallkeule quer zu ihrer Ausbreitungsrichtung keinerlei mechanisch bewegte Teile erforderlich sind.

Für eine ggf. erwünschte sektorförmige Abtastung des Objekts 1 ist erfindungsgemäß auch eine Vorrichtung vorgesehen, vergl. Fig. 4, mittels derer der Ultraschallwandler 3 über den abzutastenden Bereich quer zu der Schallkeule 31 schwenkbar ist.

Eine vorteilhafte Weiterbildung der Erfindung sieht vor, daß zum Wechsel der Abtastpositionen quer zu der Schallkeule eine Vielzahl von in Reihe angeordneten Ultraschallwandlern derart angeordnet ist, daß sie durch eine an sich bekannte Schaltungsanordnung gleichzeitig oder zeitlich nacheinander aktivierbar sind und einen Sektor des abzutastenden Objekts 1 abdecken. Dazu kann erfindungsgemäß, vergl. Fig. 5, entweder vorgesehen sein, daß die Ultraschallwandler $3_1'...3_n'$ auf einem bogenförmigen Tragkörper 6 befestigt sind, so daß die Schallkeulen $31_1'...31_n'$ radial zueinander ausgerichtet sind, und daß die einzelnen Ultraschallwandler $3_1'...3_n'$ zeitlich nacheinander durch gleichförmige Impulse aktiviert werden oder daß, vergl. Fig. 6,

0049518

80 P 7575 E

die Ultraschallwandler $3_1''...3_n''$ auf einem ebenen Tragkörper 7 befestigt sind und daß die einzelnen Ultraschallwandler $3_1''...3_n''$ zum Erzielen unterschiedlicher Strahlrichtungen zum Zwecke einer Sektor-Abtastung jeweils in ihrer Gesamtheit, jedoch mit Impulsen verschiedener Phasenlagen derart erregt werden, daß sich die jeweils erforderliche Strahlrichtung ergibt.

Auch diese zuletzt genannten Vorrichtungen bieten den Vorteil, daß keinerlei mechanisch bewegte Teile für die Wege der Schallkeule quer zu ihrer Ausbreitungsrichtung zum Zwecke einer sektorförmigen Abtastung des Objekts erforderlich sind.

13 Patentansprüche

6 Figuren

Patentansprüche:

1. Verfahren zum Abtasten eines Objekts zu dessen Abbildung mittels Ultraschall-Echo-Tomographie, d a d u r c h   g e k e n n z e i c h n e t , daß das abzubildende Objekt (1) jeweils aus verschiedenen Einstrahlrichtungen (2) mittels zumindest eines Ultraschallwandlers (3), der eine schmale, den jeweils abzutastenden Bereich des Objekts (1) nur teilweise erfassende Schallkeule (31) aufweist, schrittweise nacheinander aus einer Vielzahl von Abtastpositionen, nämlich nach Art eines Scan-Vorganges, abgetastet wird, wobei der Wechsel der Abtastpositionen quer zu der Schallkeule (31) orientiert ist, und daß die Echofolgen aus jeder der Abtastpositionen eines solchen Scan-Vorganges aufsummiert werden und somit die Wirkung einer breiten Schallkeule erreicht wird.

2. Verfahren nach Anspruch 1. d a d u r c h g e k e n n z e i c h n e t , daß die Echofolgen aus jeder der Positionen analog aufsummiert werden.

3. Verfahren nach Anspruch 1, d a d u r c h g e k e n n z e i c h n e t , daß die Echofolgen aus jeder der Positionen digital aufsummiert werden.

4. Verfahren nach einem der vorhergehenden Ansprüche, d a d u r c h g e k e n n z e i c h n e t , daß die Echofolgen aus jeder der Positionen vor ihrer Summation jeweils analog gleichgerichtet werden.

5. Verfahren nach einem der Ansprüche 1-3, d a d u r c h g e k e n n z e i c h n e t , daß die Echofolgen aus jeder der Positionen vor ihrer Summation digital gleichgerichtet werden.

6. Verfahren nach Anspruch 1, d a d u r c h
g e k e n n z e i c h n e t , daß der Wechsel der
Abtastpositionen quer zu der Schallkeule einer
lateralen Bewegung folgt.

7. Verfahren nach Anspruch 1, d a d u r c h
g e k e n n z e i c h n e t , daß der Wechsel der
Abtastpositionen quer zu der Schallkeule einer
Schwenkbewegung folgt.

8. Abtastvorrichtung zur Durchführung des Verfahrens
nach den Ansprüchen 1 und 6, d a d u r c h
g e k e n n z e i c h n e t , daß zu dem Wechsel
der Abtastpositionen quer zu der Schallkeule eine
Verschiebeanordnung (4) vorgesehen ist, mittels
derer der Ultraschallwandler (3) schrittweise
relativ zu dem abzutastenden Objekt (1) verschiebbar
ist.

9. Abtastvorrichtung zur Durchführung des Verfahrens
nach den Ansprüchen 1 und 6, d a d u r c h
g e k e n n z e i c h n e t , daß zu dem Wechsel
der Abtastpositionen quer zu der Schallkeule eine
Vielzahl von in Reihe angeordneten Ultraschallwandlern ($3_1 \ldots 3_n$) vorgesehen ist und daß die
einzelnen Ultraschallwandler ($3_1 \ldots 3_n$) dieser
Reihenanordnung in an sich bekannter Weise zeitlich
nacheinander aktiviert werden.

10. Abtastvorrichtung zur Durchführung des Verfahrens
nach den Ansprüchen 1 und 7, d a d u r c h
g e k e n n z e i c h n e t , daß zu dem Wechel
der Abtastpositionen quer zu der Schallkeule der
Ultraschallwandler (3) schwenkbar angeordnet ist
und daß der Ultraschallwandler (3) das Objekt (1)
sektorförmig abtastet.

0049518
80 P 7 5 7 5 E

11. Abtastvorrichtung zur Durchführung des Verfahrens nach den Ansprüchen 1 und 7, d a d u r c h g e k e n n z e i c h n e t , daß zu dem Wechsel der Abtastpositionen quer zu der Schallkeule eine Vielzahl von in Reihe angeordneten Ultraschallwandlern ($3_1'...3_n'$) derart angeordnet ist, daß sie durch eine an sich bekannte Schaltungsanordnung (5) gleichzeitig oder zeitlich nacheinander aktivierbar sind und einen Sektor des abzutastenden Objekts (1) abdecken.

12. Abtastvorrichtung nach Anspruch 11, d a d u r c h g e k e n n z e i c h n e t , daß die Ultraschallwandler ($3_1'...3_n'$) auf einem bogenförmigen Tragkörper (6) befestigt sind, so daß die Schallkeulen ($31_1'...31_n'$) radial zueinander ausgerichtet sind, und daß die einzelnen Ultraschallwandler ($3_1'...3_n'$) zeitlich nacheinander durch gleichförmige Impulse aktiviert werden.

13. Abtastvorrichtung nach Anspruch 11, d a d u r c h g e k e n n z e i c h n e t , daß die Ultraschallwandler ($3_1''...3_n''$) auf einem ebenen Tragkörper (7) befestigt sind und daß die einzelnen Ultraschallwandler ($3_1''...3_n''$) zum Erzielen unterschiedlicher Strahlrichtungen zum Zwecke einer Sektor-Abtastung jeweils in ihrer Gesamtheit, jedoch mit Impulsen verschiedener Phasenlagen derart erregt werden, daß sich die jeweils erforderliche Strahlrichtung ergibt.

FIG 1

FIG 3

FIG 2

FIG 4    FIG 5    FIG 6

Europäisches Patentamt

**EUROPÄISCHER RECHERCHENBERICHT**

| | EINSCHLÄGIGE DOKUMENTE | | KLASSIFIKATION DER ANMELDUNG (Int. Cl.) |
|---|---|---|---|
| Kategorie | Kennzeichnung des Dokuments mit Angabe, soweit erforderlich der maßgeblichen Teile | betrifft Anspruch | |
| X | DE - B2 - 2 618 178 (TOKYO SHIBAURA)<br><br>* Fig. 1-4,8; Spalte 5, Zeile 37 - Spalte 8, Zeile 55; Spalte 10, Zeile 44 - Spalte 11, Zeile 57; Patentansprüche 1,2,5 *<br><br>-- | 1,2,4, 6,9 | A 61 B 10/00<br>G 01 N 29/00 |
| X | US - A - 4 099 419 (HITACHI MEDICAL)<br><br>* Fig. 1a,1b,1c,2; Spalte 1, Zeile 33 - Spalte 3, Zeile 24 *<br><br>-- | 1,7,11 13 | |
| X | DE - A1 - 2 637 283 (THE COMMON-WEALTH OF AUSTRALIA)<br><br>* Fig. 1,2; Seite 10, 2. Absatz - Seite 11, 1. Absatz; Patentansprüche 1,2,8,9 *<br><br>-- | 1,7,10 11,12 | **RECHERCHIERTE SACHGEBIETE (Int. Cl.)**<br><br>A 61 B 10/00<br>G 01 N 29/00<br>G 01 N 29/04 |
| X | DE - A1 - 2 619 231 (THE COMMON-WEALTH OF AUSTRALIA)<br><br>* Fig. 1,4; Seite 17, letzter Absatz - Seite 19, 1. Absatz; Patentansprüche 9, 11 *<br><br>-- | 1,7,10 11,12 | |
| X | DE - A - 2 329 387 (THE COMMON-WEALTH OF AUSTRALIA)<br><br>* Fig. 2,3; Seite 5, 2. Absatz - Seite 10, 1. Absatz *<br><br>-- | 1,7,10 11,12 | **KATEGORIE DER GENANNTEN DOKUMENTE**<br><br>X: von besonderer Bedeutung<br>A: technologischer Hintergrund<br>O: nichtschriftliche Offenbarung<br>P: Zwischenliteratur |
| X | AT - B - 354 777 (THE COMMON-WEALTH OF AUSTRALIA)<br><br>* Fig. 1-4; Seite 3, Zeile 45 - Seite 4, Zeile 36 *<br><br>-- | 1,6,7, 8,10, 11,12 | T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| WIEN | 08-01-1982 | LUDWIG |

EPA form 1503.1   06.78

Europäisches
Patentamt

**EUROPÄISCHER RECHERCHENBERICHT**

| | EINSCHLÄGIGE DOKUMENTE | | KLASSIFIKATION DER ANMELDUNG (Int. Cl.³) |
|---|---|---|---|
| Kategorie | Kennzeichnung des Dokuments mit Angabe, soweit erforderlich, der Maßgeblichen Teile | betrifft Anspruch | |
| X | US - A - 3 439 530 (FLAHERTY et al.)<br><br>* Fig. 5-7; Spalte 7, Zeile 54 - Spalte 9, Zeile 66 *<br><br>-- | 1,7,10 | |
| X | US - A - 3 480 002 (FLAHERTY et al.)<br><br>* Fig. 1,2; Spalte 3, Zeile 61 - Spalte 5, Zeile 34 *<br><br>-- | 1,6,7,8,10 | |
| | US - A - 3 856 985 (TOKYO SHIBAURA)<br><br>* Fig. 1,2; Spalte 3, Zeile 19 - Spalte 4, Zeile 15; Spalte 8, Zeilen 24-44 *<br><br>-- | 1,3,5,6,8 | RECHERCHIERTE SACHGEBIETE (Int. Cl.³) |
| | DE - B1 - 2 543 678 (TOKYO SHIBAURA)<br><br>* Fig. 1; Spalte 5, Zeilen 10-26 *<br><br>-- | 1,2 | |
| A | J. und H. KRAUTKRÄMER "Werkstoff-prüfung mit Ultraschall", 1961<br><br>SPRINGER-VERLAG, Berlin, Göttingen, Heidelberg<br>Seiten 165,166<br><br>* Seite 165, 2. Absatz von unten; Abb. 2.29c *<br><br>---- | 4 | |